# FASCICULE DE BREVET EUROPEEN

(11) **EP 2 194 777 B1**
(45) Date de publication et mention de la délivrance du brevet: **31.10.2012**
(21) Numéro de dépôt: 08829954.0
(22) Date de dépôt: 12.09.2008
(51) Int. Cl.: A01K 67/033

(54) **PROCEDE DE TRAITEMENT PAR LUTTE BIOLOGIQUE**
BEHANDLUNGSVERFAHREN MITTELS BIOLOGISCHER BEKÄMPFUNG
TREATMENT METHOD USING BIOLOGICAL CONTROL

(30) Priorité: 14.09.2007 EP 07116463
(43) Date de publication de la demande: 16.06.2010
(73) Titulaire: Université de Liège, 4000 Liège (BE)
(72) Inventeur: LEROY, Pascal, B-1030 Bruxelles (BE); CAPELLA, Quentin, B-6220 Fleurus (BE); VERHEGGEN, François, B-5030 Gembloux (BE); FRANCIS, Frédéric, B-4300 Waremme (BE); HAUBRUGE, Eric, 1457 Walhain (BE)
(74) Mandataire: Bounaga, Sakina
(86) Numéro de dépôt international: PCT/EP2008/062195
(87) Numéro de publication internationale: WO 2009/034178

(56) Documents cités:
- EP-A- 1 161 863
- FR-A1- 2 852 937
- GB-A- 2 305 843
- US-A- 4 418 647
- US-A- 5 799 607
- US-A1- 2005 178 337

## Description

### Domaine de l'invention

L'invention porte sur l'utilisation d'un dispositif de prévention et de lutte contre les insectes ravageurs de végétaux.

### Etat de la technique

Les aphidiens représentent un groupe important de ravageurs en agriculture et en horticulture dans le monde. On en distingue pas moins de 4000 espèces (Dixon A.F.G. (1998). Aphid ecology, an optimization approach. Second edition. Chapman and Hall, London. pp 300) qui causent des dommages importants à la plante-hôte selon des mécanismes d'action divers.

Les pucerons sont des insectes de petite taille (1 à 4 millimètres) exclusivement phytophages, appartenant à l'ordre des Homoptères (Homoptera) (Dixon, 1998). Ils sont pourvus d'un rostre leur permettant de sucer la sève circulant dans les tissus conducteurs des plantes-hôtes, ainsi que d'une paire d'antennes leur permettant de capter les odeurs telles que les phéromones d'alarme et sexuelles (Pickett et al., (1992). Annual Review of Entomology 37, 69-70). L'abdomen des pucerons présente deux cornicules (protubérances tubulaires) qui peuvent excréter une substance cireuse permettant de coller les pièces buccales d'un éventuel ennemi, mais aussi émettre des phéromones d'alarme telles que le (E)-β-farnésène, afin d'avertir les autres pucerons de la présence d'un danger (Herrbach, (1992) J. of Plant Pathol. 98 (2), 62-71).

En se nourrissant de la sève et donc des éléments nutritifs nécessaires au bon développement de la plante, les pucerons causent des dégâts directs en entraînant un affaiblissement et une perturbation de la croissance du végétal-hôte. Ainsi, les piqûres et l'injection de salive ont une action irritante et toxique qui conduit même à la déformation des feuilles.

On distingue aussi deux types de dégâts indirects : l'excrétion du miellat qui est l'excrément liquide des pucerons, et la propagation de virus phytopathogènes. Le miellat, substance sucrée et riche en acides aminés, offre un milieu idéal au développement d'organismes fongiques saprophytes (fumagines) qui, réduisant la transpiration et la photosynthèse, affectent la croissance et le développement de la plante (dessèchement, chute prématurée de feuilles, ...). Un autre effet indirect est la transmission de certains virus phytopathogènes entraînant l'apparition de nombreuses maladies chez les plantes-hôtes (CAB International (1996). Crop Protection Compendium - Global module, 2nd Edition).

De nombreux moyens de lutte contre ce type de ravageurs ont déjà été décrits et font communément appel à des molécules insecticides (CN1068473).

Cependant, des méthodes alternatives de lutte existent en réponse à une résistance accrue des ravageurs aux insecticides chimiques. Elles mettent en jeu des pièges attractifs pour les ravageurs, des diffuseurs contenant des substances répulsives (US 5,792,467), des microorganismes (KR20040006457), des ennemis naturels (US 6,544,513) et des sémiochimiques (US 2005/249,769 ; US 6,890,525) - molécules naturellement impliquées dans la communication entre individus - parmi lesquels on distingue deux groupes : les *phéromones* et les substances *allélochimiques* (Nordlund et Lewis, (1976). Journal of Chemical Ecology 2 (2), 211-220).

En plus de la lutte contre les ravageurs, certaines méthodes permettent aussi la *prévention* contre des ravageurs tels que les pucerons (CN1593101). Celles-ci font alors appel soit à des insecticides (JP6192014), soit à des solutions à base de microorganismes (CN1068473). D'autres techniques proposent encore d'organiser les cultures de telle manière à promouvoir la présence d'ennemis naturels (DE4115493).

De nombreux prédateurs et parasitoïdes à différents stades de leur développement (larves, pupes, momies, adultes) sont déjà utilisés actuellement pour lutter contre les pucerons (ils sont alors aphidiphages) et d'autres ravageurs. Ces ennemis naturels des ravageurs tels que les pucerons sont classiquement désignés par le terme « auxiliaires » (en anglais, « beneficials »). Le document US-A-4,418,647 décrit l'utilisation d'une membrane artificielle pour induire l'oviposition et ensuite le développement in vitro de Trichogramma.

Actuellement, les méthodes d'utilisation d'auxiliaires reposent :
- soit sur leur placement au champ ou sur la plante par le dépôt de larves d'auxiliaires sur la plante infestée (par exemple *Adalia bipunctata, Chrysopa carnea,* ...) ;
- soit sur le lâcher inondatif d'insectes adultes susceptibles de pondre des oeufs dont il est attendu qu'ils se transformeront en larves capables de se nourrir des ravageurs ;
- soit sur le dépôt de pupes (par exemple *Episyrphus balteatus, Aphidoletes aphidimyza,* ...) ou de momies (par exemple *Aphidius colemani, Aphidius ervi,* ...) d'insectes prédateurs.

En particulier, il est bien connu que les larves d'auxiliaires comme celles de chrysopes (par exemple *Chrysopa carnea, Chrysoperla lucasina,* ...), de coccinelles (par exemple *Adalia bipunctata, Harmonia axyridis,* ...) et de syrphes (*Episyrphus balteatus, Eupeodes corollae,* ...) se nourrissent d'insectes ravageurs d'un végétal comme, par exemples, les pucerons (aphidiens - *Myzus persicae, Acyrthosiphon pisum, Aphis fabae,* ... -), les cochenilles (par exemple *Planococcus citri*), l'aleurode (par exemple *Trialeurodes vaporariorum, Bemisia tabaci*) ou encore les psylles (par exemple *Psylla pyri*).

Selon leur stade de développement (adultes, larves, pupes, momies), les auxiliaires sont conditionnés en vue de leur utilisation au champ ou à proximité du végétal à traiter.

Plusieurs inconvénients sont liés à l'utilisation d'auxiliaires selon ces méthodes :
- le dépôt des larves est contraignant et requiert du temps : il faut placer les larves sur le végétal ;
- le délai de livraison des larves doit être très bref : en absence de proies, la plupart des larves se dévorent l'une l'autre (cannibalisme) ;
- le lâcher d'adultes ne garantit pas que ces derniers vont agir *in-situ ;* en effet, la plupart d'entre eux sont mobiles et capables de quitter la zone ciblée ;
- le dépôt de momies ou de pupes donne lieu à des adultes qui peuvent également quitter la zone ciblée ;
- la production des larves, des momies et des adultes est coûteuse : tout le cycle de développement doit être assuré en laboratoire. Il faut donc des proies (pucerons) qui se développent sur des plantes-hôtes : leur maintenance est donc onéreuse et requiert une main d'oeuvre considérable.

La présente invention a donc pour objet de remédier à au moins un des inconvénients cités ci-dessus.

### Bref résumé de l'invention

La présente invention selon la revendication 1 a pour but de résoudre les problèmes techniques liés aux méthodes de lutte contre des ravageurs de plantes et de cultures au moyen d'auxiliaires en utilisant un dispositif capable de produire, au départ d'oeufs, des larves se nourrissant d'insectes ravageurs de plantes et ce, de façon simple, reproductible et peu coûteuse.

Le procédé de traitement par lutte biologique proposé ici permet d'introduire des oeufs au moment opportun et en proportion adéquate selon les infestations de ravageurs.

En particulier, la présente invention selon la revendication 1 concerne l'utilisation d'un dispositif pour prévenir l'apparition d'insectes ravageurs de plantes ou pour lutter contre ces ravageurs.

Le dispositif peut par exemple se présenter sous forme d'un récipient dans lequel la ponte par un insecte prédateur a été stimulée au moyen d'agents de stimulation de ponte (ou agents d'oviposition) en l'absence à la fois de pucerons et de végétal. Le dispositif peut également se présenter sous forme d'une lamelle, de bille, de poudre, de copeau ou de fibre, couverte d'au moins un agent de stimulation de ponte. La lamelle, bille, poudre, copeau ou fibre peut être de n'importe quel matériau sélectionné parmi le groupe de matériaux listés ci-après.

Le dispositif contient donc sur au moins une de ses surfaces des oeufs pondus par une femelle d'un insecte prédateur d'un insecte ravageur de plante. Selon un mode de réalisation du dispositif, la femelle d'un insecte prédateur est enfermée momentanément dans le dispositif ou placé à proximité du dispositif en présence d'un agent de stimulation de ponte avant d'en être retirée en fin de ponte. Selon un autre mode de réalisation du dispositif, la femelle d'un insecte prédateur est placée momentanément dans le dispositif ou placé à proximité du dispositif en présence d'un agent de stimulation de ponte avant que le dispositif ne soit récupéré en fin de ponte.

Selon l'invention, lesdits oeufs sont choisis parmi le groupe comprenant les oeufs d'insectes appartenant à l'ordre des Neuroptera, des Coleoptera et des Diptera. De préférence, que lesdits oeufs sont choisis parmi le groupe comprenant les oeufs d'insectes appartenant aux familles des Syrphidae, Coccinellidae ou Chrysopidae. Par exemple, lesdits oeufs ou larves issues desdits oeufs peuvent être choisis parmi le groupe comprenant les oeufs ou larves issues desdits oeufs d'insectes appartenant au genre *Episyrphus, Chrysopa, Harmonia,* et *Adalia,* tels que *Adalia bipunctata, Chrysopa carnea, Chrysoperla lucasina, Episyrphus balteatus, Aphidoletes aphidimyza, Eupeodes corollae* et *Harmonia axyridis.*

Selon un mode de réalisation particulier, lesdits insectes ravageurs appartiennent à l'ordre des Homoptera. Selon un mode de réalisation particulier, lesdits insectes ravageurs sont des pucerons, des cochenilles, des aleurodes, des psylles ou appartiennent à la famille des Aphidae.

Selon un mode de réalisation particulier, ledit au moins un agent de stimulation de ponte est choisi parmi le groupe comprenant les :
- les alcools aliphatiques et aromatiques, aldéhydes, esters, lactones ;
- les monoterpènes ;
- les sesquiterpènes et leurs alcools, aldéhydes et dérivés d'esters associés
- le miellat naturel ou artificiel ; ou
- un mélange de ceux-ci, optionnellement en solution ou en suspension dans un solvant organique.

Selon un mode de réalisation particulier, ledit au moins un agent de stimulation de ponte est choisi parmi le groupe des composés suivants : linalool, 3-hexanol, hexanol, 1-octen-3-ol, 3-hexenol, 2-hexenol, hexanal, 2-nonenol, 2-nonenal, γ-terpineol, 2-phenylethanol, nepetalactone, nepetalactol, α-pinene, β-pinne, limonene, 3-carene, 2-carene, α-phellandrene, β-phellandrene, m-cymene, germacrene, (E)-β-farnesène (EBF), (Z)-β-farnesène (E-E)-α-farnesène, (E-Z)-α-farnesène, (Z-Z)-α-farnesène, (Z-E)-α-farnesène, α-humulene, β-caryophyllene, leur alcools, aldéhydes et dérivés d'esters, le miellat naturel ou artificiel ; ou un mélange de ceux-ci.

Le dispositif peut aussi comprendre en outre des agents de conservation ou de nutrition desdits oeufs et larves.

L'invention concerne donc l'utilisation d'un dispositif pour prévenir l'apparition d'insectes ravageurs de plantes ou pour lutter contre ces ravageurs. Cette utilisation est particulièrement bien adaptée contre les insectes ravageurs appartenant à l'ordre des Homoptera, tels que par exemple les insectes ravageurs appartenant à la famille des Aphidae tels que les pucerons, ou encore, des insectes ravageurs tels que les cochenilles, les aleurodes ou les psylles.

Un mode de réalisation particulier comprend les étapes suivantes :
- placer au moins un dispositif directement sur la plante à protéger ou à traiter, ledit dispositif comprenant au moins une surface inerte et libre de tout insecte ravageur et au moins un agent de stimulation de ponte, ladite surface comprenant des oeufs d'au moins un insecte prédateur dudit ravageur;
- assurer un contact avec le végétal afin de permettre aux larves issues des oeufs de se déplacer en direction des ravageurs constituant ses proies.

D'autres aspects, particularités et avantages de la présente invention apparaîtront à la lecture de la description qui va suivre et des exemples qui l'illustrent.

### Brève description des figures

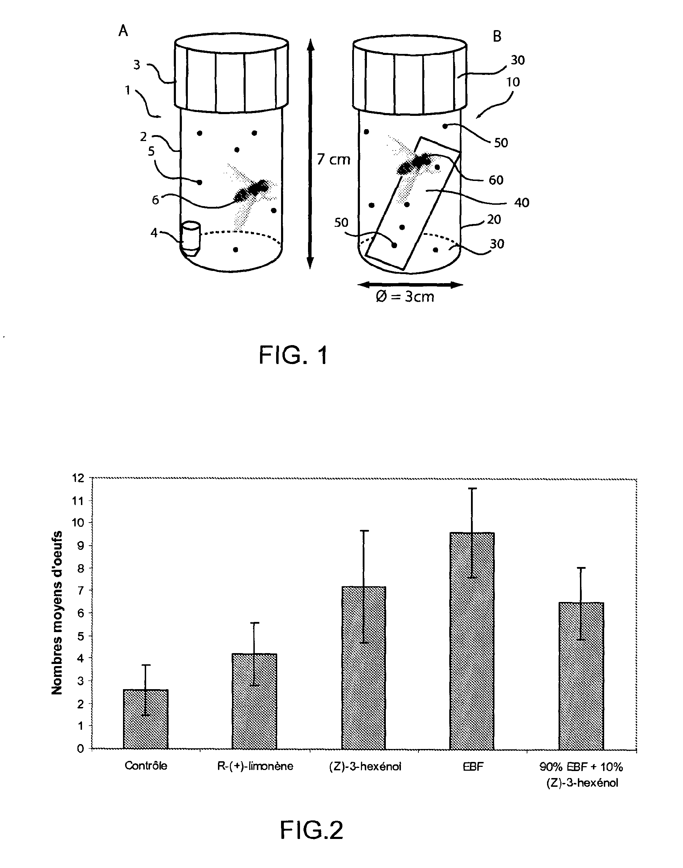

La figure 1 illustre schématiquement deux systèmes A et B utilisés pour la préparation de dispositifs selon des modes de réalisation particuliers obtenus.
La figure 2 représente un graphe donnant le nombre moyens d'oeufs (+/- SE) pondus par des femelles d'*E. balteatus* (2 à 4 semaines d'âge / n=10) durant 3 heures dans des dispositifs selon des modes de réalisation particuliers (volume = 50 cm³) selon qu'elles sont en présence de composés sémiochimiques utilisés seuls ou en mélange. Les moyennes de mesures obtenues avec les sémiochimiques ou les mélanges de sémiochimiques sont significativement différentes par rapport au contrôle (à P<0,05-Tests de Dunnett, α=0.05).

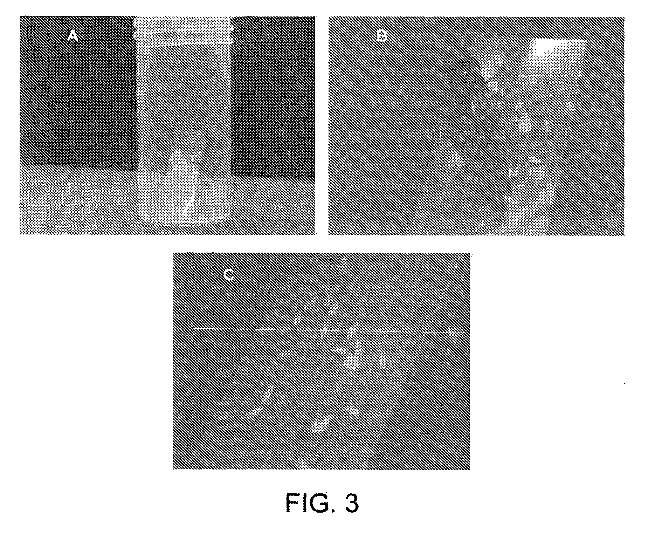

La figure 3 présente trois photographies d'un dispositif selon un mode de réalisation particulier de la présente invention. La photographie A montre une vue globale du dispositif. Les photographies B et C montrent des vues plus détaillées du dispositif de la photographie A.

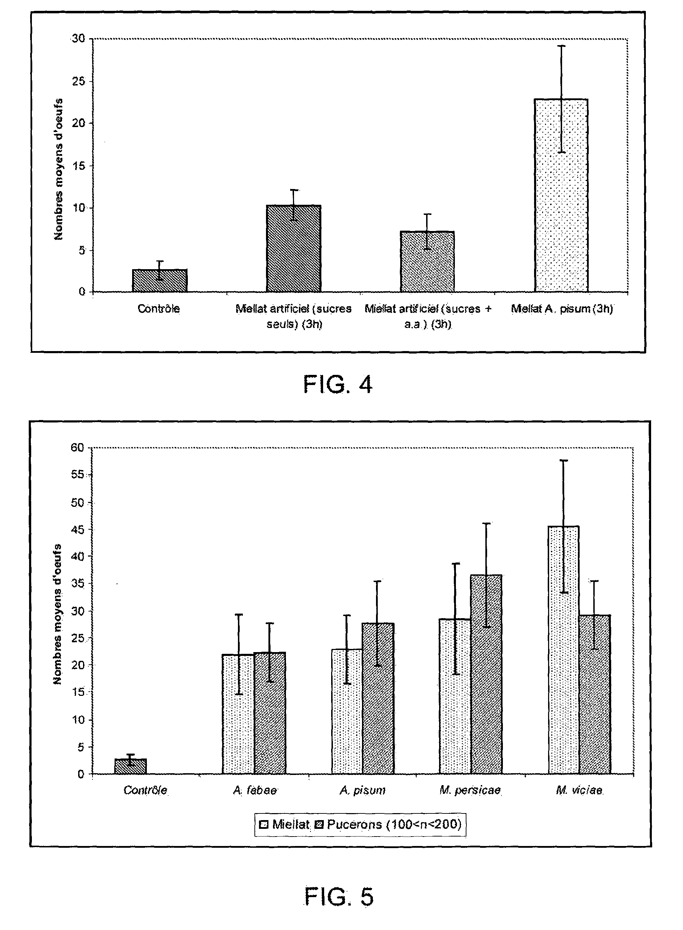

La figure 4 représente un graphe donnant le nombre moyens d'oeufs (+/- SE) pondus par des femelles d'E. *balteatus* (2 à 4 semaines d'âge / n=10) durant 3 heures dans des piluliers (volume = 50 cm³) selon qu'elles sont en présence de miellat collecté sous des plants de *Vicia faba* infestés de pucerons (*Acyrthosiphon pisum*) ou de miellats artificiels.
Les moyennes de mesures obtenues avec le miellat naturel et les miellats artificiels sont significativement différentes par rapport au contrôle (à P<0,05- Tests de Dunnett, α=0.05).
La figure 5 représente un graphe donnant le nombre moyens d'oeufs (+/- SE) pondus par des femelles d'E. *balteatus* (2 à 4 semaines d'âge / n=10) durant 3 heures dans des piluliers (volume = 50 cm³) selon qu'elles sont en présence de miellat naturel (collecté sous des plants de *Vicia faba* infestés de pucerons de différentes espèces) ou bien en présence de diverses espèces de pucerons. Les moyennes de mesures obtenues avec le miellat naturel et les pucerons sont significativement différentes par rapport au contrôle (à P<0,05- Tests de Dunnett, α=0.05).

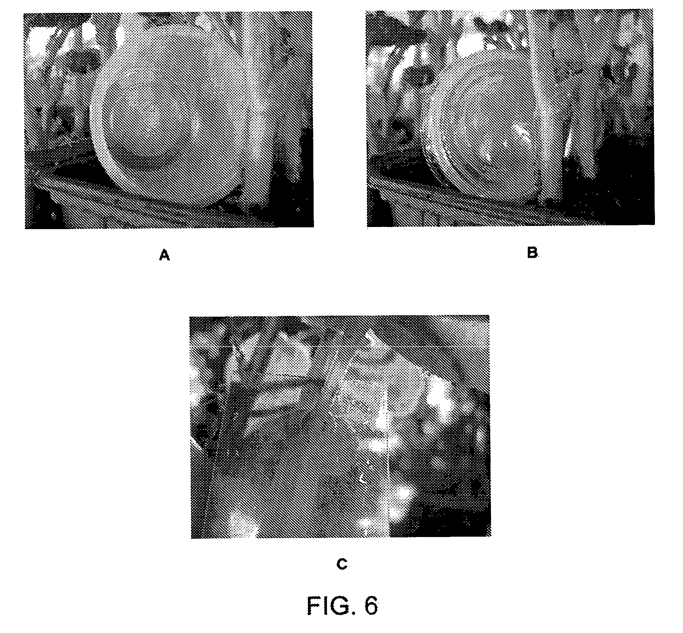

La figure 6 présente trois photographies montrant différentes formes du dispositif de lutte biologique comportant des oeufs d'*E*. *balteatus* : (A) pilulier à ouverture complète, (B) pilulier à ouverture partielle et (C) lamelle.

### Description détaillée de l'invention

L'invention porte sur l'utilisation d'un dispositif de prévention et de lutte contre les insectes ravageurs de végétaux, comme par exemple les aphidiens tels que les pucerons.

Le dispositif contient donc sur au moins une de ses surfaces des oeufs pondus par au moins une femelle d'un insecte prédateur d'un insecte ravageur de plante. Une des surfaces dudit dispositif est inerte et dépourvue d'insectes ravageurs. Le terme « inerte » se réfère à une surface dont la matière n'est pas vivante ou « surface de matériau non-vivant ».

Lors de la préparation du dispositif, au moins une femelle dudit insecte prédateur est enfermée momentanément dans le dispositif ou placée à proximité du dispositif (dans le cas où les pontes se font dans une pièce plus grande ou dans une cage où seraient placés les dispositifs) en présence d'un agent de stimulation de ponte avant d'en être retirée en fin de ponte. Les dispositifs peuvent être des récipients, des lamelles, des copeaux, des fibres, de la poudre ou des billes par exemple.

Par exemple, une ou plusieurs femelles peuvent être enfermées dans une enceinte dans laquelle passent ou séjournent durant un temps déterminé les supports de ponte (par exemple le ou les récipients, lamelles, billes, poudres, fibres, copeaux, ...), après la ponte les supports avec les oeufs sont enlevés de l'enceinte, par exemple via une bande transporteuse, et ensuite sont distribués sur les plantes par divers procédés tel que par pulvérisation à sec, dans un liquide, etc.

Le dispositif comprend au moins une surface comprenant des oeufs d'insectes prédateurs, encore appelés « auxiliaires » ou « ennemis naturels », par exemples des oeufs de coccinelles, syrphes, chrysopes, ..., et éventuellement ou dans certains cas des larves issues de ces oeufs. Ce dispositif est caractérisé en ce qu'il comprend en outre des agents de stimulation de ponte des oeufs par l'insecte prédateur (appelé agent d'oviposition ou agent stimulant) et optionnellement au moins une surface de ponte et optionnellement des agents de conservation ou de nutrition des larves issues desdits oeufs.

Selon un mode de réalisation particulier, le dispositif et/ou ladite surface inerte est choisi parmi le groupe comprenant un récipient, une lame, des fibres, des copeaux, des poudres, une lamelle, ou des billes, etc. Le dispositif peut être de n'importe quelle forme tel qu'un flacon, boîte, tube, sachet, lamelle, feuille de plastique, poudre, billes..., ou de n'importe quelle taille.

De préférence, le dispositif est composé d'un récipient (ou contenant ou réceptacle) dans lequel ou sur lequel sont disposés des oeufs et des agents de stimulation de ponte.

Il peut être fermé ou ouvert par tout moyen adapté à la forme et à la taille du dispositif et en fonction de la nécessité. Il peut lui-même constituer une surface de ponte ou comprendre au moins une surface de ponte indépendante (support de ponte). De préférence, ladite surface de ponte est sélectionnée parmi le groupe comprenant une lame, une lamelle, des poudres, des copeaux, des fibres ou des billes.

Avantageusement, le dispositif est une récipient, une lamelle, des billes, des copeaux, des poudres ou des fibres contenant ou comportant des oeufs d'insectes prédateurs, et contenant en outre un ou plusieurs agents de stimulation de ponte.

Selon un mode de réalisation particulier, quand le dispositif est un récipient, ce dernier est de préférence muni d'un système de fermeture et d'ouverture ou d'un autre dispositif d'obturation susceptible de fermer ou d'ouvrir le récipient en fonction de la nécessité. Par exemple, le récipient peut être sélectionné parmi le groupe comprenant un tube, un sachet, un pilulier, un flacon, une boîte munis d'un couvercle ou d'un autre dispositif d'obturation susceptible de fermer ou d'ouvrir le récipient en fonction de la nécessité.

Le système de fermeture et d'ouverture dudit dispositif peut être un cordon, une fermeture zip, un couvercle ou tout autre système de fermeture adapté au dit dispositif.

Le matériau du dispositif (ledit dispositif pouvant être sélectionné parmi un récipient, une lamelle, des billes, des copeaux, des poudres ou des fibres) est de préférence inerte et peut être de différentes natures telles que du verre, plastique, bois, papier, carton, métal, polymère, ... ou un matériau issu de leur combinaison, ou tout autre matériau adapté à la réalisation du dispositif.

Préférentiellement le matériau dudit dispositif est un polymère organique tel que le polyéthylène (PE), le polypropylène (PP), le chlorure de polyvinyle (PVC), le polycarbonate, le polyamide, le polystyrène (PS), .... Plus préférentiellement un matériau biodégradable. Parmi les matériaux ainsi définis, on trouve le carton, le papier ou les polymères dégradables tels que l'acide polyglycolique (PGA), l'acide polylactique (PLA), les polylactones tels que le polycaprolactone (PCL), les esters de polyalkylenes, les esters de polyamides, les esters de polyvinyle, les alcools de polyvinyle, les polyanhydrides, ....

Selon un autre mode de réalisation particulier ledit dispositif est une lame ou lamelle.

Selon un autre mode de réalisation particulier ledit dispositif comprend des billes ou est constitué de billes. Les billes peuvent être de différentes natures telles que du verre, plastique, bois, papier, carton, métal, ... ou un matériau issu de leur combinaison, ou tout autre matériau adapté à la réalisation du présent dispositif.

Préférentiellement le matériau des billes est un polymère organique tel que le polyéthylène (PE), le polypropylène (PP), le chlorure de polyvinyle (PVC), le polycarbonate, le polystyrène (PS), .... Plus préférentiellement, le matériau des billes est un matériau biodégradable. Parmi les matériaux ainsi définis, on trouve le carton, le papier ou les polymères dégradables tels que l'acide polyglycolique (PGA), l'acide polylactique (PLA), les polylactones tels que le polycaprolactone (PCL), les esters de polyalkylenes, les esters de polyamides, les esters de polyvinyle, les alcools de polyvinyle, les polyanhydrides, ....

Des billes ou tout autre forme adéquate d'alginate, de chitosan ou de toute autre nature peuvent aussi être utilisées.

Les billes comportant des oeufs peuvent être placées ou non dans une solution aqueuse ou huileuse avant leur placement sur le terrain par pulvérisation ou toute autre technique d'application. Un enrobage des billes ou leur mise en suspension dans un liquide aqueux ou huileux doit permettre leur « fixation » sur le végétal. Des tensioactifs ou n'importe quelle autre technique peuvent être utilisés afin de permettre aux billes de se « fixer » sur le végétal lors de leur utilisation.

Selon un autre mode de réalisation particulier ledit dispositif comprend ou est constitué de copeaux ou de sciure. Les copeaux ou la sciure peuvent être de différentes natures telles que du plastique, bois, papier, carton, métal, ... ou un matériau issu de leur combinaison, ou tout autre matériau adapté à la réalisation du présent dispositif. De préférence, le dispositif comprend ou est constitué de copeaux ou de sciure de bois.

Selon un autre mode de réalisation particulier ledit dispositif comprend ou est constitué de fibres. Les fibres peuvent être composées de matériaux organiques et inorganiques, le matériau pouvant être un non tissé, un tissu voire même une grille. Comme fibre organique entrant dans la constitution des fibres, on utilise de préférence mais de façon non limitative, les fibres naturelles, en particulier de cellulose, de coton, les fibres chimiques organiques, en particulier de cellulose modifiée, de méthylcellulose, de rayonne, d'acrylique, de nylon, de polyester, de polyéthylène, de polypropylène, de polyamide. Les fibres inorganiques sont en particulier des fibres de verre ou de métal.

Selon un autre mode de réalisation particulier ledit dispositif comprend ou est constitué de poudres ou granules. Par « poudre ou granule », on entend en particulier toute composition, sous forme de particules ou granules de toutes granulométries, s'écoulant généralement librement. Le matériau des poudres ou granules peut être sélectionné parmi le verre, le bois, le métal, les polymères d'origine synthétique tels que les polyvinylalcools, les polyvinylpyrrolidones, les (co)-polymères d'acide (méth) acrylique et leurs sels, les polyéthylène glycols, les polyesters de préférence biodégradables comme les polylactates, les polycaprolactones, les polyesters de di (ou poly) acides et de di (poly) alcools et les polymères d'origine naturelle ou fermentaire tels que les xanthanes, les polyhydroxyalcanoates (PHA), les amidons, les celluloses, les protéines et leurs dérivés respectifs.

Les oeufs et les larves issues de cesdits oeufs contenus dans le récipient ou se trouvant sur un support de ponte sont des oeufs et larves d'insectes prédateurs utilisés comme ennemis naturels (encore dénommés auxiliaires) de ravageurs de végétaux.

On appelle auxiliaire ou ennemi naturel tout être vivant qui par son mode de vie entraîne l'inhibition ou la destruction d'espèces nuisibles à l'agriculture ou l'horticulture. Il s'agit de tout prédateur qui, par son mode de vie, apporte son concours à la destruction de ravageurs nuisibles aux cultures.

Plus particulièrement les oeufs ou les larves issues desdits oeufs concernés par la présente invention sont issus de femelles gravides appartenant à l'ordre des Neuroptera, des Coleoptera et des Diptera. Plus particulièrement ils sont issus des femelles gravides appartenant aux familles des Syrphidae, Coccinellidae ou Chrysopidae.

En particulier, la (les) femelle(s) gravide(s) peut (peuvent) appartenir à l'espèce *balteatus* du genre *Episyrphus* (famille des Syrphidae et ordre Diptera) ou encore à l'espèce *carnea* du genre *Chrysopa* (famille des Chrysopidae et ordre Neuroptera), ou encore à l'espèce *axyridis* du genre *Harmonia* (famille des Coccinellidae et ordre Coleoptera), ou encore à l'espèce *bipunctata* du genre *Adalia* (famille des Coccinellidae et ordre Coleoptera), ou encore à l'espèce *septempunctata* du genre *Coccinella* (famille des Coccinellidae et ordre Coleoptera).

De façon préférée le dispositif est réalisé en utilisant la (les) femelle(s) gravide(s) de *Episyrphus balteatus* ou de *Eupeodes corollae* (également appartenant à la famille des Syrphidae).

On appelle ravageur ou insecte ravageur : tout être vivant qui, par son action et son mode de vie, affecte la croissance des végétaux, ce qui entraîne des pertes économiques et de rendement.

Les ravageurs concernés par la présente invention appartiennent essentiellement à l'ordre des Homoptera et à la famille des Aphidae.

Les espèces appartenant à la famille des Aphidae et concernées par la présente invention sont notamment mais de façon non limitative *Acyrthosiphon pisum, Myzus persicae, Aphis fabae* et *Megoura viciae.*

Des ravageurs autres que les aphidiens sont également visés par la présente invention, notamment d'autres Homoptères comme les cochenilles (par exemple l'espèce *Planococcus citri*), l'aleurode (par exemple les espèces *Trialeurodes vaporariorum, Bemisia tabaci*) ou encore les psylles (par exemple l'espèce *Psylla pyri*).

De préférence un composé sémiochimique ou un mélange de composés sémiochimiques ou un miellat naturel ou artificiel sont choisis parmi les agents de stimulation de la ponte par la femelle gravide de l'espèce de prédateur choisi.

Au sens de la présente invention, on appelle composé sémiochimique tout composé affectant le comportement de certains insectes parmi lesquels on distingue deux groupes: les phéromones et les substances allélochimiques. Les substances allélochimiques sont des métabolites secondaires émis par un individu d'une espèce et capables d'affecter la santé, la croissance, le comportement et/ou la biologie d'un individu d'une autre espèce. On y retrouve les kairomones, les allomones et les synomones (Arnaud et al., 2003 Journal Des Ingénieurs 87 : 25-28). Les phéromones sont des substances sécrétées par un individu et captées par un autre individu de la même espèce.

Un grand nombre de composés sémiochimiques notamment d'origine végétale ou aphidienne et les métabolites secondaires bien connus de l'homme de l'art conviennent pour la réalisation du dispositif. Ces composés peuvent être utilisés seuls ou en mélange.

Les composés ou des compositions sémiochimiques d'origine aphidienne ou végétale qui conviennent pour la réalisation du dispositif incluent :
- des alcools aliphatiques et aromatiques, aldéhydes, esters, lactones (notamment le linalool, 3-hexanol, hexanol, 1-octen-3-ol, 3-hexenol, 2-hexenol, hexanal, 2-nonenol, 2-nonenal, γ-terpineol, 2-phenylethanol, nepetalactone, nepetalactol) ;
- des monoterpènes tels que l'α-pinene, β-pinene, limonene, 3-carene, 2-carene, α-phellandrene, β-phellandrene, m-cymene, germacrene ;
- des sesquiterpènes et leurs alcools, aldéhydes et dérivés d'esters associés tels que l'α-humulene, le β-caryophyllene, le (E)-β-farnesene (EBF) et ses dérivés comme les isomères (Z)-β-farnesène (E-E)-α-farnesène, (E-Z)-α-farnesène, (Z-Z)-α-farnesène, (Z-E)-α-farnesène.

Le miellat naturel d'origine aphidienne et ses produits de dégradation ainsi que le miellat artificiel reproduisant au mieux le miellat naturel peuvent également être utilisés.

On entend par miellat naturel un terme générique définissant les excréments liquides des Homoptères. Ce rejet métabolique comprend essentiellement des sucres - 90 à 95% de la matière sèche - (monosaccharides - fructose, galactose, glucose, mannose, ... -, disaccharides - maltose, melibiose, sucrose, tréhalose, turanose, ... - et trisaccharides - fructomaltose, mélézitose, raffinose, ... -) (Yao & Akimoto, 2001. Oecologia, 128 : 36-43, Wäckers 2005 Plant-provided Food for Carnivorous Insects (ed. by FL Wäckers, PCJ van Rijn & J Bruin), pp. 17-74. Cambridge University Press, Cambridge, UK)), des acides aminés libres, des protéines, des minéraux, des vitamines, des lipides et des acides organiques (Way, 1963. Annual Review of Entomology, 8: 307-344; Buckley, 1987, Annual Review of Ecology and Systematics, 8: 111-135).

Plus particulièrement le miellat produit par le(s) puceron(s) colonisant une plante peut être collecté et disposé sur un support solide en vue de l'introduire dans le dispositif. Des exemples sont repris dans la partie « Exemples ». Cependant, l'utilisation d'un miellat artificiel est préférée comme l'utilisation de sucres seuls ou des mélanges de sucres ou encore des solutions miellées avec ou sans ajout d'acides aminés. L'utilisation d'un miellat artificiel permet d'éliminer le maintien coûteux d'un élevage de ravageurs.

On entend par miellat artificiel une solution aqueuse de sucres comme notamment : glucose, fructose, tréhalose, saccharose, .... Il peut contenir en outre des acides aminés choisis parmi n'importe quel acide aminé naturel ou non-naturel, des sels organiques ou inorganiques, des lipides, des vitamines ou des minéraux, ainsi que des agents de conservation. En général, les miellats artificiels consistent en un mélange de sucrose, de levure (autolysat de levure ou extrait de levure) et d'eau. Par exemple, une solution à base de miel, de tryptophane et de levure, ces trois composants étant présentés seuls ou en association, peut être utilisée comme miellat artificiel.

Le choix du composé ou de la composition sémiochimique pour réaliser le dispositif sera opéré en fonction de l'espèce de prédateur sélectionné pour la ponte des oeufs et plus particulièrement en fonction de son effet d'oviposition efficace auprès de la femelle gravide de l'espèce sélectionnée dans le dispositif ou à proximité du dispositif. Le choix du composé ou de la composition sémiochimique approprié en fonction du prédateur peut être opéré au cas par cas par l'homme de l'art qui dispose de nombreux documents de l'état de l'art à cet effet.

Selon un mode de réalisation particulier, le composé sémiochimique peut être fourni dans le dispositif dans ou sur un support. Ledit support peut être un diffuseur ou une lame ou lamelle, un fil, un morceau de tissu, un tampon, de la poudre, des copeaux, des fibres ou des billes, ... sur lequel est disposé une petite quantité - de l'ordre de quelques µg à quelques g - du composé ou de la composition sémiochimique et dont la nature peut être du verre, un polymère organique, un polymère organique biodégradable, du papier, du carton ou du métal. Il peut également être un gel.

Les agents de conservation qui peuvent optionnellement être introduits dans le dispositif, dans ou sur le support sont des composés ou des compositions bien connus de l'homme de l'art permettant d'éviter l'altération des incitants à la ponte, par exemple par des moisissures, et ainsi le maintien des oeufs dans des conditions optimales.

On entend par agents de nutrition, des composés ou des mélanges aptes à permettre la survie et la croissance des larves issues des oeufs pondus. Ces agents de nutrition peuvent être sélectionnés parmi les mélanges bien connus de l'homme de l'art, notamment du pollen, du sucrose et des extraits de levure.

La figure 1 illustre schématiquement deux dispositifs selon des modes de réalisation particuliers. Les dispositifs 1,10 sont composés de récipients 2,20. Le récipient 2,20 peut aussi être pourvu d'un système de fermeture 3,30. Les récipients 2,20 des dispositifs 1,10 contiennent des oeufs 5,50 d'insectes prédateurs. Lors de la préparation des dispositifs, les oeufs sont pondus par une femelle gravide 6,60 enfermée dans lesdits dispositifs 1,10. Selon un mode de réalisation particulier, le dispositif 1 de la figure 1A comprend un diffuseur 4 d'agents de stimulation de ponte (non montré). Selon un autre mode de réalisation particulier, le dispositif 10 de la figure 1B comprend une lamelle 40 revêtue d'agent de stimulation de ponte des oeufs 50.

Une méthode pour réaliser le dispositif comprend les étapes suivantes :
a. disposer dans ou sur le dispositif, au moins un agent de stimulation de ponte (par exemple sélectionné parmi un composé sémiochimique ou un mélange de composés sémiochimiques ou un miellat naturel ou artificiel), ledit agent de stimulation de ponte peut être disposé tel quel dans le dispositif ou fourni dans un diffuseur ou sur un support susceptible d'être mis en forme (feuille de plastique, gel, billes, poudres, copeaux, etc) ;
b. placer à l'intérieur ou à proximité dudit dispositif au moins une femelle gravide issue des familles de prédateurs sensibles à l'effet d'oviposition développé par ledit au moins un agent de stimulation de ponte disposé à l'étape (a) ;
c. attendre le temps nécessaire pour que au moins une femelle gravide ponde des oeufs. Le temps peut varier et peut s'étaler typiquement de quelques minutes à plusieurs heures, voire plusieurs jours ;
d. en fin de ponte, la au moins une femelle est soustraite au milieu de ponte et optionnellement conservée pour une utilisation ultérieure ; ou alternativement le dispositif est retiré du milieu de ponte ;
e. optionnellement, la méthode comprend aussi l'introduction dans le dispositif d'éléments de conservation des oeufs et des éléments nutritifs des larves.

Selon un mode de réalisation particulier, la méthode comprend l'enfermement de ladite femelle à l'intérieur dudit dispositif, le dispositif étant ensuite obturé. Selon un mode de réalisation particulier de la méthode, le dispositif est un récipient.

Selon un autre mode de réalisation particulier, la méthode comprend :
a. disposer dans un récipient (pilulier, tube à essai muni d'un couvercle vissable, sachet, boîte, ...) une surface de ponte (de quelque nature que ce soit) sur laquelle est disposé au moins un agent de stimulation de la ponte pour la femelle gravide de l'espèce de prédateur choisi ;
b. introduire dans le dispositif au moins une femelle gravide issue des familles de prédateurs et sensible à l'effet d'oviposition développé par le composé ou le mélange sémiochimique de l'étape (a) ;
c. obturer le récipient au moyen d'un système de fermeture quelconque ;
d. attendre le temps nécessaire pour que au moins une femelle gravide ponde des oeufs soit sur ladite surface de ponte, soit à n'importe quel endroit du récipient ou les deux. Le temps peut varier et peut s'étaler typiquement de quelques minutes à plusieurs heures, voire plusieurs jours ;
e. en fin de ponte, la au moins une femelle est soustraite au milieu de ponte et optionnellement conservée pour une utilisation ultérieure dans un dispositif similaire ou identique ;
f. optionnellement, la méthode comprend aussi l'introduction d'éléments de conservation des oeufs et des éléments nutritifs des larves.

Selon un autre mode de réalisation particulier, la méthode comprend :
a. pulvériser/enduire une lamelle avec au moins un composé sémiochimique ou un mélange de composés sémiochimiques ou un miellat naturel ou artificiel choisi préférentiellement parmi des agents de stimulation de la ponte par la femelle gravide de l'espèce de prédateur choisi ;
b. placer/suspendre la lamelle dans un emplacement fermé (par exemple une cage ou une pièce) en présence de nombreuses femelles gravides issues des familles de prédateurs et sensibles à l'effet d'oviposition développé par le composé ou le mélange sémiochimique de l'étape (a) ;
c. attendre le temps nécessaire pour que les femelles gravides pondent des oeufs sur ladite lamelle. Le temps peut varier et peut s'étaler typiquement de quelques minutes à plusieurs heures, voire plusieurs jours ;
d. en fin de ponte, la lamelle couverte d'oeufs est récupérée et placée dans un contenant quelconque avant le placement sur le terrain.

Selon un autre mode de réalisation particulier, la méthode comprend :
a. pulvériser/enduire des billes avec au moins un composé sémiochimique ou un mélange de composés sémiochimiques ou un miellat naturel ou artificiel choisi préférentiellement parmi des agents de stimulation de la ponte par la femelle gravide de l'espèce de prédateur choisi ;
b. placer les billes dans un emplacement fermé, par exemple une cage ou une pièce, en présence de nombreuses femelles gravides issues des familles de prédateurs et sensibles à l'effet d'oviposition développé par le composé ou le mélange sémiochimique de l'étape (a) ;
c. attendre le temps nécessaire pour que les femelles gravides pondent des oeufs sur les billes. Le temps peut varier et peut s'étaler typiquement de quelques minutes à plusieurs heures, voire plusieurs jours ;
d. en fin de ponte, les billes couvertes d'oeufs sont récupérées et placées dans un contenant quelconque avant le placement sur le terrain.

Comme décrit ci-dessus, les lamelles ou les billes peuvent être de n'importe quelle matière et de n'importe quelle forme.

Le dispositif est utilisé pour prévenir l'apparition de ravageurs, en particulier les aphidiens, sur n'importe quel végétal, plante ou culture agricole ou horticole, ou pour lutter contre ces ravageurs lorsqu'ils infestent ce végétal, plante ou culture. On entend par plante tout végétal ou culture agricole ou horticole.

Selon un mode de réalisation particulier, l'utilisation du dispositif comprend :
- placer le dispositif à proximité de ou directement sur la plante à protéger ou à traiter soit en le fixant à proximité ou sur une partie de la plante par quelque moyen que ce soit ou en le déposant à même le sol ou sur un support à proximité de la cible (plante, végétal) ;
- assurer une ouverture du dispositif et/ou un contact avec le végétal afin de permettre aux larves issues des oeufs de se déplacer en direction des ravageurs constituant ses proies. Cette étape peut aussi consister à pulvériser les oeufs sur le végétal en assurant une adhérence des billes (solution aqueuse, ...).

Une seconde forme d'utilisation comprend le retrait, du dispositif, de la surface ou support de ponte sur laquelle se trouvent les oeufs et de la fixer ou de la déposer à proximité de la plante ou du végétal à protéger ou à traiter.

Une troisième forme d'utilisation, si les oeufs sont obtenus sur des billes ou tout autre support adapté, placé ou non en solution aqueuse ou huileuse, est de pulvériser ces billes sur le végétal en utilisant n'importe quelle technique adéquate. L'enrobage des billes doit permettre à celles-ci de se fixer sur une partie de la plante. Les substances d'enrobage peuvent être toutes les substances qui permettent aux billes de se fixer sur une partie de la plante.

Le nombre, l'emplacement et la fréquence d'utilisation des dispositifs ou de la (des) surface(s) de ponte qui en est (sont) retirée(s) et sur laquelle (lesquelles) se trouvent des oeufs seront déterminés par l'utilisateur en fonction du degré d'infestation du végétal, de la plante ou de la culture, de l'efficacité aphidiphage des larves et du nombre d'oeufs contenus dans les dispositifs.

Avantageusement, le dispositif comprend au minimum 1 oeuf, de préférence au minimum 2, au minimum 3 oeufs, de préférence au minimum 4 oeufs, au minimum 5, 6, 7, 8, 9, 10, 11, 12, 13, 14, 15, 16, 17, 20, 25, ou au minimum 30 oeufs.

Les dispositifs ou la surface de ponte sur laquelle se trouvent des oeufs sont soit enlevés en fin d'opération afin d'être réutilisés ultérieurement ou encore abandonnés lorsqu'ils sont constitués d'un matériau biodégradable.

Lorsque le dispositif ou la surface de ponte qui en est extraite est expédié depuis son lieu de préparation vers le lieu d'utilisation, le colis d'expédition peut également contenir quelques pucerons mais préférentiellement des agents de nutrition afin d'éviter tout danger de cannibalisme entre les larves dans le cas ou un retard d'expédition devrait déjà permettre l'émergence d'un nombre important de larves.

Un procédé de production, et de conditionnement d'oeufs d'insectes prédateurs comprenant les étapes suivantes :
(a) la disposition sur une surface (par exemple la surface d'une lamelle, d'une bille, de copeaux, de poudres, de fibres, d'un récipient ou un support ou surface de ponte contenu dans ledit récipient) d'au moins un agent de stimulation de ponte, par exemple un composé sémiochimique ou un mélange de composés sémiochimiques ou un miellat naturel ou préférentiellement artificiel pouvant être choisi préférentiellement parmi les agents de stimulation de la ponte ;
(b) la ponte d'oeufs par au moins une femelle gravide d'insecte prédateur sur ladite surface; et
(c) le retrait après la ponte de ladite ou des femelle(s) et/ou la récupération de la surface comprenant les oeufs.

Contrairement aux méthodes de production de larves de prédateurs décrites dans l'état de l'art qui reposent,
- souvent, sur l'utilisation de pucerons comme source d'agent d'induction de ponte et
- toujours, sur l'utilisation d'une partie d'un végétal comme support de ponte,
le dispositif permet de produire en masse des oeufs de prédateurs rapidement et sans recourir ni à l'utilisation de pucerons, ni à l'utilisation d'une partie de végétal comme support de ponte. On dispose ainsi, sur un support inerte, d'oeufs pouvant être conservés quelques jours à basses températures avant leur placement au moment opportun sur le terrain.

Les exemples ci-dessous démontrent que le dispositif contient des oeufs de prédateurs sans avoir recours ni à des pucerons, ni à des plantes comme support de ponte.

Il en découle une production (i) rapide, en quantité et contrôlée (par exemple, une femelle *d'Episyrphus balteatus* pond, en moyenne, une trentaine d'oeufs en quelques heures, par exemple en 3h), et (ii) moins coûteuse puisqu'elle ne nécessite plus l'élevage coûteux de pucerons et permet de limiter la main d'oeuvre (il suffit de placer une femelle en présence des incitants de ponte et de collecter les oeufs après quelque temps, typiquement quelques heures).

Contrairement à une surface de ponte végétale susceptible de se dégrader, le récipient ou le support non-vivant (inerte) (tel que plastique, carton, papier, verre, gel, etc ...) contenu dans celui-ci présente une résistance accrue dans le temps et dans les conditions d'utilisation réelle (température, ensoleillement, ...).

Par rapport aux utilisations de larves prédatrices décrites dans l'état de l'art dont le dépôt sur plante est contraignant et requiert du temps, le placement au champ du dispositif est aisé et rapide. Si le matériau du dispositif est choisi parmi les matériaux biodégradables, le dispositif ne doit plus être récupéré en fin d'opération de lutte contre les ravageurs ce qui constitue un avantage supplémentaire.

Contrairement aux larves produites loin de l'endroit à traiter ou à protéger, les larves produites par le dispositif vont émerger à l'endroit précis de la cible et entamer directement leur action prédatrice permettant donc d'optimiser le potentiel prédateur de la larve. La présente invention permet l'introduction au moment voulu et en quantité voulue d'oeufs de prédateurs sur le terrain afin de renforcer la présence d'auxiliaires et d'assurer une lutte biologique optimale face aux infestations de ravageurs. L'introduction d'oeufs sur le terrain permet, finalement, après l'action prédatrice des larves et l'élimination des ravageurs, l'obtention d'adultes. Ces adultes, une fois gravides, peuvent pondre naturellement et renforcer la présence d'auxiliaires in-situ.

Notons également que, s'en référant à l'état de l'art, les larves produites à distance de la plante à traiter sont conditionnées puis expédiées au champ. Il est donc communément admis que le nombre de larves expédiées ne peut pas être trop élevé sous peine de risquer leur destruction par cannibalisme. De la même manière, le temps d'expédition doit être bref pour assurer le maximum de survie parmi les larves. Ces problèmes sont levés par le dispositif puisque le dispositif expédié contient essentiellement des oeufs : après la ponte, les larves apparaissent après 4 à 5 jours.

Les exemples suivants sont donnés à titre illustratif et non limitatif, susceptibles de variantes aisément accessibles à l'homme de l'art.

### Exemples

### Exemple 1 : Préparation de dispositifs selon des modes de réalisation partit pour les prédateurs Episyrphus balteatus (Diptera : Syrphidae), Chrysopa carnea (Neuroptera : Chrysopidae) et Adalia bipunctata (Coleoptera : Coccinelidae)

### 1.a Méthode

La préparation des dispositifs, et les pontes (oviposition) décrites ci-dessous ont été réalisées dans une chambre conditionnée (Température (Temp) = 20 ± 1°C ; HR (humidité relative) = 60 ± 10% ; 16h de photopériodisme). Les pontes d'oeufs ont été réalisées dans un récipient en plastique (pilulier d'un volume de 50cm³) (tel que schématiquement illustré aux figures 1A et 1B).
- Dans un premier mode de réalisation, on a introduit dans un récipient un diffuseur contenant :
   1. des composés volatils : EBF, (Z)-3-hexénol et R-(+)-limonène, seuls ou en mélange ;
   2. de l'huile de paraffine comme contrôle.

Chaque sémiochimique ou le mélange de sémiochimiques a été dosé à 40 µg puis déposé dans le diffuseur (100 µl d'une solution à 400 ng/ µl dont le solvant est l'huile de paraffine).

Pour chacun des cas testés, une femelle gravide d'*E*. *balteatus,* de *C*. *carnea* ou de *A. bipunctata* a été sélectionnée aléatoirement.
- Dans un second mode de réalisation, on a disposé à l'intérieur d'un récipient préalablement à l'introduction de la femelle gravide, une languette en plastique supportant du miellat artificiel.

Du miellat naturel, produit par le puceron *Acyrthosiphon pisum,* a été récolté sur des feuilles de plastique placées sous des plants de *Vicia faba* infestés de pucerons avant d'être découpées en languettes (1 x 5 cm). La préparation de lamelles avec du miellat naturel constitue le témoin positif. Des lamelles sans aucun composant inducteur de ponte constituent le contrôle.

Dans un premier temps, deux compositions de miellat artificiel ont été testées : (i) une solution sucrée (37% de glucose + 32% de fructose + 9% de tréhalose + 2% de saccharose), (ii) la même solution sucrée à laquelle sont ajoutés les 4 acides aminés principaux du miellat (0.05% d'asparagine ; 0.05% de glutamine ; 0.02% de glutamate ; 0.02% de sérine).

Dans un second temps, deux autres compositions de miellat artificiel ont été testées : (iii) une solution miellée (50%) et (iv) un mélange dans des proportions 2 :1 de solution miellée (50%) et de solution d'(E)-β-farnésène (400 ng/µL) dans de l'huile de paraffine. Ces solutions sont pulvérisées sur les languettes de plastique.

Pour les deux modes de réalisation (diffuseur avec sémiochimique(s) et languette avec miellat artificiel), les femelles testées (n=10) âgées de 2 à 4 semaines ont été isolées de tout site d'oviposition 24 heures avant les expériences proprement dites. Les durées de ponte sont de 3 à 5h pour *E*. *balteatus* et de 12 à 24h pour *C*. *carnea* et *A. bipunctata.*

### 1.b.1. Récipient contenant un diffuseur contenant des sémiochimiques :

Des oeufs ont été pondus par les femelles *d'Episyrphus balteatus,* de *Chrysopa carnea* et *d'Adalia bipunctata* dans le dispositif contenant des sémiochimiques placés dans un diffuseur. La ponte par induction à l'aide de composés volatils a eu lieu en l'absence de pucerons et de leurs plantes-hôtes. Pour le syrphe *Episyrphus balteatus* (figure 2), un nombre moyen de 9.6 ± 2.0 oeufs a été pondu en réponse à l'EBF, 7.2 ± 2.5 en réponse au (Z)-3-hexénol, 4.2 ± 1.4 oeufs en réponse au R-(+)-limonène et 6.5 ± 1.6 oeufs en réponse au mélange 90% EBF + 10% (Z)-3-hexénol. Des résultats similaires ont été obtenus pour la coccinelle *Adalia bipunctata* et le chrysope *Chrysopa carnea.* Le nombre d'oeufs pondus, dans des piluliers avec un diffuseur contenant des sémiochimiques, est toujours significativement plus élevé par rapport au nombre d'oeufs obtenu avec le contrôle (à P<0,05- Tests de Dunnett, a=0.05), sauf pour le R-(+)-limonène.

Les résultats de cet exemple ont montré que les femelles *d'Episyrphus balteatus,* de *Chrysopa carnea* et *d'Adalia bipunctata* sont capables de pondre en l'absence de pucerons et de végétal, dans des piluliers où un diffuseur contient des sémiochimiques, permettant ainsi d'obtenir un dispositif avec un grand nombre d'oeufs. Les sémiochimiques utilisés seuls ou en mélange pour le dispositif induisent la ponte des femelles même en l'absence de végétal et de pucerons.

### 1.b.2. Récipient contenant une languette couverte de miellat artificiel à base de sucres et d'acides aminés :

Le miellat naturel a été récolté sur une feuille en plastique disposée sous un plant de *Vicia faba* infesté de pucerons afin de constituer le témoin positif. Les miellats artificiels selon les compositions décrites dans le paragraphe 1.a. de l'exemple 1 (i et ii) ont été pulvérisés sur des languettes de plastiques lesquelles sont ensuite disposées dans le pilulier.

Les feuilles couvertes de fines gouttes de miellat artificiel ont alors été découpées en de petites languettes (1 x5cm) placées dans les piluliers (figure 1B et figure 3A, B et C).

Les résultats ont montré que la ponte, induite à l'aide de miellat artificiel à base de sucres et d'acides aminés, a eu lieu en l'absence de pucerons et de leurs plantes-hôtes. Pour le syrphe *Episyrphus balteatus* (figure 4), un nombre moyen de 10.3 ± 1.8 en réponse au mélange de sucres (glucose + fructose + tréhalose + saccharose) et 7.2 ± 2.1 oeufs en réponse au mélange de sucres et d'acides aminés (glucose + fructose + tréhalose + saccharose + asparagine + glutamine + glutamate + sérine). Des résultats similaires ont été obtenus pour la coccinelle *Adalia bipunctata* (35.3 ± 8.1 oeufs pour la solution de sucres seuls et 37.6 ± 3.4 oeufs pour la solution de sucres avec les acides aminés) et le chrysope *Chrysopa carnea* (17.0 ± 2.1 oeufs pour la solution de sucres seuls et 22.0 ± 1.6 oeufs pour la solution de sucres avec les acides aminés).

Un grand nombre d'oeufs a été pondu par femelles *d'Episyrphus balteatus*, de *Chrysopa carnea* et *d'Adalia bipunctata* dans et sur les surfaces du récipient et sur la languette contenant le miellat artificiel à base de sucres et d'acides aminés, permettant ainsi d'obtenir un dispositif avec un grand nombre d'oeufs. Le nombre d'oeufs pondus, dans des piluliers contenant une languette couverte de miellat artificiel à base de sucres et d'acides aminés, est toujours significativement plus élevé par rapport au nombre d'oeufs obtenu avec le contrôle (à P<0,05- Tests de Dunnett, α=0.05).

### 1.b.3. Récipient contenant une languette couverte de miellat artificiel à base de solutions miellées et d'(E)-β-farnésène

Le miellat naturel a été récolté sur une feuille en plastique disposée sous un plant de *Vicia* faba infesté de pucerons afin de constituer le témoin positif. Les miellats artificiels selon les compositions décrites dans le paragraphe 1.a. de l'exemple 1 (iii et iv) ont été pulvérisés sur des languettes de plastiques lesquelles sont ensuite disposées dans le pilulier.

Les feuilles couvertes de fines gouttes de miellat artificiel ont alors été découpées en de petites languettes (1 x5cm) placées dans les piluliers (figure 1B et figure 3A, B et C).

Les résultats ont montré que la ponte, induite à l'aide de miellat artificiel à base de solutions miellée et d'(E)-β-farnésène, a eu lieu en l'absence de pucerons et de leurs plantes-hôtes. Pour le syrphe *Episyrphus balteatus,* un nombre moyen de 14.8 ± 2.9 oeufs a été pondu en réponse au mélange solution miellée + EBF (2 :1) et de 10.0 ± 2.9 oeufs en réponse à la solution miellé (50%). Des résultats similaires ont été obtenus pour la coccinelle *Adalia bipunctata* (33.1 ± 3.1 oeufs pour la solution miellée avec de l'(E)-β-farnésène et 28.0 ± 2.2 oeufs pour la solution miellée seule) et le chrysope *Chrysopa carnea* (19.0 ± 0.8 oeufs pour la solution miellée avec de l'(E)-β-farnésène et 16.0 ± 1.2 oeufs pour la solution miellée seule).

Le nombre d'oeufs pondus, dans des piluliers contenant une languette couverte de miellat artificiel à base solutions miellées et d'(E)-β-farnésène, est toujours significativement plus élevé par rapport au nombre d'oeufs obtenu avec le contrôle (à P<0,05- Tests de Dunnett, α=0.05).

### Exemple 2 : Comparaison de l'effet de différents miellats naturels ou de la présence de pucerons, sur l'oviposition d'Episyrphus balteatus, Chrysopa carnea (Neuroptera : Chrysopidae) et Adalia bipunctata (Coleoptera : Coccinelidae) dans la préparation d'un dispositif selon un mode de réalisation particulier.

Différentes espèces de pucerons produisant le miellat (*A. fabae, M. persicae, M. viciae* et *A. pisum*) ont été utilisées. Dix femelles (2 à 4 semaines d'âge) ont été testées pour chaque espèce de pucerons et chaque miellat naturel (Temp = 20 ± 1°C ; HR = 60 ± 10% ; 16h de photopériodisme). Le nombre d'oeufs déposés a été relevé après 3h. Les résultats sont montrés à la figure 5 pour le syrphe *Episyrphus balteatus.* Les résultats montrent que quelle que soit l'origine du miellat, ce dernier induit toujours l'oviposition chez le syrphe E, *balteatus* dans un récipient tel que celui de l'exemple 1 (pilulier).

Des résultats similaires ont été obtenus pour *Chrysopa carnea* et *Adalia bipunctata :* la présence de miellat seul permet toujours l'obtention d'au moins autant d'oeufs que ceux obtenus lors de la présence de pucerons.

En comparant la présence de miellat à celle de pucerons pour chacune des espèces de pucerons envisagées et pour chaque prédateur (syrphe, coccinelle et chrysope), on constate que le miellat stimule la ponte et donne lieu à un nombre d'oeufs au moins aussi élevé que celui enregistré lors de la présence de pucerons.

Cette expérience montre que la présence de pucerons et de végétal n'est pas indispensable à l'oviposition des syrphes, des coccinelles et des chrysopes et qu'un dispositif peut être obtenu grâce à la méthode comprenant l'utilisation d'un inducteur de ponte (agent sémiochimique) dans un récipient pour induire la ponte de l'insecte prédateur sur un support inerte.

### Exemple 3 : Utilisation du dispositif comportant des oeufs d'Episyrohus balteatus (Diptera Syrphidae) ou de Chrysopa carnea (Neuroptera : Chrysopidae) ou d'Adalia bipunctata (Coleoptera : Coccinelidae) pour lutter contre des ravageurs

### 3.a. Méthode

Afin de tester l'efficacité du dispositif de lutte biologique, des plants de fève (*Vicia faba*) (15-20 cm) ont été infestés avec une quantité connue de pucerons (*Acyrthosiphon* pisum ou *Aphis fabae*). Suite au placement du dispositif de lutte biologique à proximité ou sur le végétal, un suivi des colonies de pucerons et du nombre de larves d'*Episyrphus balteatus* ou de *Chrysopa carnea* ou *d'Adalia bipunctata* présentes sur le végétal a été assuré. Parallèlement à ces essais, un contrôle a toujours été mis en place : sans placement du dispositif de lutte biologique, il s'agit d'un plant de fève infesté d'une quantité de pucerons identique à celle présente sur les plants-tests (avec le dispositif de lutte biologique). L'efficacité du dispositif de lutte biologique a été mesurée, quotidiennement, en comparant le nombre de pucerons présents sur le contrôle au nombre de pucerons présents sur les plants-tests (avec le dispositif de lutte biologique).

L'obtention des oeufs dans/sur le dispositif de lutte biologique (lamelle) a été réalisée selon les techniques et les modalités décrites précédemment.

Une première série d'essais a été menée en *conditions de laboratoire* (Temp = 20 ± 1 °C ; HR = 60 ± 10% ; 16h de photopériodisme) tandis qu'une seconde série d'essais a été réalisée *sur le terrain en conditions naturelles* durant les mois de juin et juillet 2008 (Temp _{moyenne} = 16,7°C / Min = 7,0°C et Max = 31,1°C ; HR_{moyenne} = 78,7 % / Min = 32,2% et Max = 99%).

### 3.b.1. Les essais en conditions de laboratoire

Au laboratoire, les essais ont porté sur trois formes de dispositif : (1) l'utilisation de *piluliers à ouverture complète* contenant 30 oeufs *d'Episyrphus balteatus* ou de *Chrysopa carnea* ou *d'Adalia bipunctata* et placés au pied du végétal (figure 6A), (2) l'emploi de *piluliers à ouverture partielle* contenant 30 oeufs *d'Episyrphus balteatus* ou de *Chrysopa carnea* ou *d'Adalia bipunctata* et placés au pied du végétal (figure 6B) et (3) l'utilisation de *lamelles* comportant un nombre variable d'oeufs *d'Episyrphus balteatus* ou de *Chrysopa* carnea ou *d'Adalia bipuntcata* (5, 10 ou 15 oeufs) sont accrochées au végétal (*Vicia faba*) (figure 6C). Les essais portant sur les différentes densités d'oeufs ont pour objectif de définir le nombre minimal d'oeufs requis pour éliminer des colonies de 200 à 300 pucerons.

Dans tous les cas de figure, les résultats montrent que le dispositif de lutte biologique est fonctionnel puisque la totalité des pucerons est éliminée après 7 à 10 jours. Toutefois, un nombre minimal de 10 à 15 oeufs *d'Episyrphus balteatus* ou de *Chrysopa carnea* ou *d'Adalia bipunctata* est requis pour éliminer des colonies de 200 à 300 pucerons (en moyenne). En moyenne, 4 à 5 larves de prédateurs (*d'Episyrphus balteatus* ou de *Chrysopa carnea* ou *d'Adalia bipunctata*) gagnent le végétal. Une différence significative du nombre de pucerons entre le contrôle (plants sans dispositif de lutte biologique) et les plants-tests (plants associés à un dispositif de lutte biologique) n'apparaît qu'après 5 à 6 jours (temps requis pour que les oeufs éclosent et que les larves entament leur consommation de ravageurs). Un nombre de 15 oeufs doit permettre d'assurer un contrôle optimal des colonies de pucerons (colonies de 300 individus).

### 3. b.2. Les essais sur le terrain (en conditions naturelles)

Sur le terrain, cinq parcelles (1m²) comportant 5 plants de fève (*Vicia faba*) ont été mises en place et infestées de pucerons (*Acyrthosiphon pisum* ou *Aphis fabae*). Le dispositif de lutte biologique sous *forme de lamelle* comportant 15 oeufs *d'Episyrphus balteatus* ou de *Chrysopa carnea* ou *d'Adalia bipunctata* a été accroché sur le végétal (figure 6C).

Ces essais montrent que le dispositif de lutte biologique est fonctionnel sur le terrain et qu'une population moyenne de 500 pucerons (au départ) est éliminée après 10 à 12 jours. En moyenne, 4 à 5 larves de prédateurs (*d'Episyrphus balteatus* ou de *Chrysopa carnea* ou *d'Adalia bipunctata*) gagnent le végétal. Une différence significative du nombre de pucerons entre le contrôle (plants sans dispositif de lutte biologique) et les plants-tests (plants associés à un dispositif de lutte biologique) n'apparaît qu'après 5 à 6 jours (temps requis pour que les oeufs éclosent et que les larves entament leur consommation de ravageurs). Un nombre de 15 à 20 oeufs doit permettre d'assurer un contrôle optimal des colonies de pucerons (colonies de 500 individus) sur le terrain en conditions naturelles.

### Exemple 4: Induction artificielle de l'oviposition à grande échelle (en masse pour Episyrphus balteatus (Diptera : Syrphidae) ou Chrysopa carnea (Neuroptera : Chrysopidae) ou Adalia bipunctata (Coleoptera : Coccinelidae) pour lutter contre des ravageurs

### 4.a. Obtention d'oeufs dans une salle conditionnée et application sur le terrain

Dans une salle conditionnée (Temp = 20 ± 1°C ; HR = 60 ± 10% ; 16h de photopériodisme) de 20m³, un élevage à grande échelle de syrphes prédateurs *Episyrphus balteatus* ou de coccinelles *Adalia bipunctata* ou de chrysopes *Chrysopa carnea* est entretenu par la disposition de plusieurs sources de nourriture et d'eau. Après le passage dans un sas d'entrée, un chariot est inséré au centre de la salle d'élevage, dont les lumières sont alors éteintes, à l'exception d'une lampe placée sous le chariot. Sur le chariot, sont disposées une série de lamelles en plastique de 5x10cm (de PVC ou de PP, ... transparente ou de couleur (jaune, verte, ...)), ou des billes ou des fibres ou des copeaux, tous ces dispositifs ou supports de ponte ayant été recouverts préalablement d'un agent de stimulation de ponte (miellat artificiel, ...).

L'oviposition est induite durant 3 heures avant que le chariot ne soit retiré de la salle d'élevage. Par la suite, les lamelles, les billes, les fibres ou les copeaux sont récoltés à l'aide par exemple d'un bande transporteuse, ... et sont placés ou pulvérisés sur les plants infestés de ravageurs appartenant à l'ordre des Homoptera, tels que les pucerons, psylles, cochenilles, ....

### 4.b. Obtention d'oeufs dans des boites d'élevage et application sur le terrain

Les insectes prédateurs, *Episyrphus balteatus, Adalia bipunctata ou Chrysopa carnea,* sont élevés au nombre de 30 dans des boites en plastique de 10x25cm, perforées d'orifices grillagés permettant l'aération. Les boites sont maintenues dans des chambres conditionnées (Temp = 20 ± 1 °C ; HR = 60 ± 10% ; 16h de photopériodisme). Du pollen de provenance florale variée et du sucre sont introduits dans des boites de Pétri ouvertes de 3cm de diamètre et des éponges imbibées permettent l'apport en eau. Tous les deux jours, une boite de Pétri de 6cm de diamètre, contenant approximativement 20 billes de chitosan (ou d'alginate) ou des lamelles, des fibres ou des copeaux, est introduite dans la boite pour une durée de 24h. Les billes ou les lamelles ou les fibres ou les copeaux sont préalablement recouverts d'un agent de stimulation de ponte (miellat artificiel, ...). Suite à la ponte, les billes ou les lamelles ou les fibres ou les copeaux recouverts d'oeufs sont récoltés (à l'aide d'un bande transporteuse, ...) et conservés jusqu'à l'application ou la pulvérisation sur les plantes infestées de ravageurs appartenant à l'ordre des Homoptera, tels que les pucerons, psylles, cochenilles, ...

Les billes ou les fibres ou les copeaux comportant les oeufs sont placés ou non dans une solution aqueuse ou huileuse avant leur placement sur le terrain par pulvérisation. Un enrobage des billes ou leur mise en suspension dans un liquide aqueux ou huileux permet leur « fixation » sur le végétal.

### 4.c. Obtention d'oeufs dans une enceinte et application sur le terrain

Les insectes prédateurs, *Episyrphus balteatus, Adalia bipunctata ou Chrysopa carnea* sont introduits dans une enceinte de quelques 10 cm de hauteur et de 1 à plusieurs m² de surface. Dans cette enceinte sont introduits des lamelles, des billes, des fibres ou des copeaux qui sont préalablement recouverts d'un agent de stimulation de ponte (par exemple miellat artificiel, ...).

Suite à la ponte, les billes, les lamelles, les fibres ou les copeaux recouverts d'oeufs sont récoltés (à l'aide d'un bande transporteuse, ...) et conservés jusqu'à l'application ou la pulvérisation sur les plantes infestées de ravageurs appartenant à l'ordre des Homoptera, tels que les pucerons, psylles, cochenilles, ...

Les billes, les fibres, les copeaux comportant les oeufs peuvent être placés dans une solution aqueuse ou huileuse avant leur placement sur le terrain par pulvérisation. Un enrobage des billes ou leur mise en suspension dans un liquide aqueux ou huileux permet leur « fixation » sur le végétal.

## Revendications

1. Utilisation en champ sur un végétal d'un dispositif pour produire des larves d'insectes prédateurs pour prévenir l'apparition d'insectes ravageurs de plantes ou pour lutter contre ces ravageurs, au moins une surface dudit dispositif étant inerte et libre de tout insecte ravageur cette surface comprenant au moins un oeuf d'au moins un insecte prédateur dudit ravageur, le dispositif comprenant au moins un agent de stimulation de ponte, lesdits oeufs étant choisis parmi le groupe comprenant les oeufs d'insectes appartenant à l'ordre des Neuroptera, des Coleoptera et des Diptera, le dispositif étant placé en contact avec le végétal afin de permettre aux larves issues des oeufs de se déplacer en direction des ravageurs constituant ses proies.

2. Utilisation selon la revendication 1, **caractérisé en ce que** lesdits oeufs sont choisis parmi le groupe comprenant les oeufs d'insectes appartenant aux familles des Syrphidae, Coccinellidae ou Chrysopidae.

3. Utilisation selon l'une quelconque des revendications 1 ou 2, **caractérisée en ce que** lesdits insectes ravageurs appartiennent à l'ordre des Homoptera.

4. Utilisation selon l'une quelconque des revendications 1 à 3, **caractérisée en ce que** lesdits insectes ravageurs sont des pucerons, des cochenilles, des aleurodes, des psylles ou appartiennent à la famille des Aphidae.

5. Utilisation selon l'une quelconque des revendications 1 à 4, **caractérisé en ce que** ledit au moins un agent de stimulation de ponte est choisi parmi le groupe comprenant les
- les alcools aliphatiques et aromatiques, aldéhydes, esters, lactones ;
- les monoterpènes ;
- les sesquiterpènes et leurs alcools, aldéhydes et dérivés d'esters associés ;
- le miellat naturel ou artificiel ; ou
- un mélange de ceux-ci.

6. Utilisation selon l'une quelconque des revendications 1 à 5 dans laquelle le dispositif comprend en outre des agents de conservation ou de nutrition desdits oeufs.

7. Utilisation selon l'une quelconque des revendications 1 à 6 dans laquelle le dispositif comprend en outre un support de ponte.

8. Utilisation selon l'une quelconque des revendications 1 à 7, **caractérisé en ce que** le dispositif et/ou ladite surface inerte est sélectionné parmi le groupe comprenant un récipient, une lame, une lamelle, des poudres, des copeaux, des fibres, ou des billes.

9. Utilisation selon la revendication 7, **caractérisé en ce que** ladite surface de ponte est sélectionnée parmi le groupe comprenant une lame, une lamelle, des poudres, des copeaux, des fibres, ou des billes.

10. Utilisation selon l'une quelconque des revendications 1 à 9, **caractérisé en ce que** le dispositif comprend au moins 1 oeuf, de préférence au moins 2 oeufs.

11. Utilisation selon l'une quelconque des revendications 1 à 10, comprenant les étapes suivantes :
- placer au moins un dispositif directement sur la plante à protéger ou à traiter ; ledit dispositif comprenant au moins une surface inerte et libre de tout insecte ravageur et au moins un agent de stimulation de ponte, ladite surface comprenant des oeufs d'au moins un insecte prédateur dudit ravageur, et
- assurer un contact avec le végétal afin de permettre aux larves issues des oeufs de se déplacer en direction des ravageurs constituant ses proies.

## Claims

1. Use in a field of a device on a plant to produce larvae of predator insects to prevent the occurrence of plant insect pests or to control said pests, at least one surface of said device being inert and free from any insect pest, said surface comprising at least one egg of at least one predator insect of said pest, the device comprising at least one egg-laying stimulating agent, said eggs being selected from the group comprising eggs of insects belonging to the orders Neuroptera, Coleoptera and Diptera, the device being placed in contact with the plant in order to permit the larvae hatching from the eggs to move in the direction of the pests which constitute its prey.

2. Use according to claim 1, **characterised in that** said eggs are selected from the group comprising eggs of insects belonging to the Syrphidae, Coccinellidae or Chrysopidae families.

3. Use according to any one of claims 1 or 2, **characterised in that** said insect pests belong to the order Homoptera.

4. Use according to any one of claims 1 to 3, **characterised in that** said insect pests are aphids, scale insects, whiteflies, psyllids or belong to the family of Aphidae.

5. Use according to any one of claims 1 to 4, **characterised in that** said at least one egg-laying stimulating agent is selected from the group comprising
- aliphatic and aromatic alcohols, aldehydes, esters, lactones;
- monoterpenes;
- sesquiterpenes and their alcohols, aldehydes and associated ester derivatives;
- natural or artificial honeydew; or
- a mixture thereof.

6. Use according to any one of claims 1 to 5, wherein the device further comprises agents for the conservation or nutrition of said eggs.

7. Use according to any one of claims 1 to 6, wherein the device further comprises an egg-laying support.

8. Use according to any one of claims 1 to 7, **characterised in that** the device and/or said inert surface is selected from the group comprising a container, a slide, a plate, powders, chips, fibres or beads.

9. Use according to claim 7, **characterised in that** said egg-laying surface is selected from the group comprising a slide, a plate, powders, chips, fibres or beads.

10. Use according to any one of claims 1 to 9, **characterised in that** the device comprises at least one egg, preferably at least two eggs.

11. Use according to any one of claims 1 to 10, comprising the following steps:
- placing at least one device directly on the plant to be protected or treated; said device comprising at least one surface which is inert and free from any insect pest and at least one egg-laying stimulating agent, said surface comprising eggs from at least one predator insect for said pest, and
- providing contact with the plant in order to permit the larvae hatching from the eggs to move in the direction of the pests which constitute its prey.

## Patentansprüche

1. Verwendung einer Vorrichtung zur Erzeugung von Raubinsektenlarven bei einer Pflanze im Freiland, um den Befall mit Pflanzenschadinsekten zu verhindern oder diese Schädlinge zu bekämpfen, wobei mindestens eine Fläche der Vorrichtung inert und frei von jedweden Schadinsekten ist, wobei diese Oberfläche mindestens ein Ei mindestens eines Insekts umfasst, welches ein Fressfeind des Schädlings ist, wobei die Vorrichtung mindestens ein Mittel der Förderung der Eiablage umfasst, wobei die Eier aus der Gruppe ausgewählt sind, die Eier von Insekten umfasst, welche zur Ordnung der Netzflügler, der Käfer und der Zweiflügler gehören, wobei die Vorrichtung derart angeordnet wird, dass sie mit der Pflanze in Kontakt ist und die Larven, welche aus den Eiern stammen, zu den Schädlingen gelangen können, welche ihre Beute darstellen.

2. Verwendung nach Anspruch 1, **dadurch gekennzeichnet, dass** die Eier aus der Gruppe ausgewählt sind, die Eier von Insekten umfasst, welche zu den Familien der Schwebfliegen, der Marienkäfer oder der Florfliegen gehören.

3. Verwendung nach einem der Ansprüche 1 oder 2, **dadurch gekennzeichnet, dass** Schadinsekten zur Ordnung der Gleichflügler gehören.

4. Verwendung nach einem der Ansprüche 1 bis 3, **dadurch gekennzeichnet, dass** die Schadinsekten Blattläuse, Schildläuse, Mottenschildläuse, Blattflöhe sind oder zur Familie der Röhrenblattläuse gehören.

5. Verwendung nach einem der Ansprüche 1 bis 4, **dadurch gekennzeichnet, dass** das mindestens eine Mittel zur Förderung der Eiablage aus der Gruppe ausgewählt ist, welche
- aliphatische und aromatische Alkohole, Aldehyde, Ester, Lactone;
- Monoterpene;
- Sesquiterpene und die dazugehörigen Alkohole, Aldehyde und Esterderivate;
- natürlichen oder künstlichen Honigtau; oder
- Mischungen derselben umfasst.

6. Verwendung nach einem der Ansprüche 1 bis 5, wobei die Vorrichtung darüber hinaus Mittel zur Erhaltung oder Ernährung der Eier umfasst.

7. Verwendung nach einem der Ansprüche 1 bis 6, wobei die Vorrichtung darüber hinaus einen Eiablageträger umfasst.

8. Verwendung nach einem der Ansprüche 1 bis 7, **dadurch gekennzeichnet, dass** die Vorrichtung und/oder die inerte Oberfläche aus der Gruppe ausgewählt ist, die einen Behälter, ein Blatt, eine Lamelle, Pulver, Schnitzel, Fasern oder Partikel umfasst.

9. Verwendung nach Anspruch 7, **dadurch gekennzeichnet, dass** die Eiablagefläche aus der Gruppe ausgewählt ist, die ein Blatt, eine Lamelle, Pulver, Schnitzel, Fasern oder Partikel umfasst.

10. Verwendung nach einem der Ansprüche 1 bis 9, **dadurch gekennzeichnet, dass** die Vorrichtung mindestens 1 Ei, vorzugsweise mindestens 2 Eier umfasst.

11. Verwendung nach einem der Ansprüche 1 bis 10, die folgenden Schritte umfassend:
- Anordnen mindestens einer Vorrichtung direkt an der zu schützenden oder zu behandelnden Pflanze; wobei die Vorrichtung mindestens eine Fläche umfasst, die inert und frei von jedweden Schadinsekten ist, sowie mindestens ein Mittel zur Förderung der Eiablage, wobei die Fläche Eier von mindestens einem Insekt umfasst, welches ein Fressfeind des Schädlings ist, und
- Sicherstellen eines Kontakts mit der Pflanze, damit die Larven, die aus den Eier stammen, zu den Schädlingen gelangen können, welche ihre Beute darstellen.
